# EUROPEAN PATENT APPLICATION

(11) **EP 4 567 409 A1**
(43) Date of publication of application: **11.06.2025**
(21) Application number: 23383264.1
(22) Date of filing: 07.12.2023
(51) Int. Cl.: G01N 21/65

(54) **MONITORING DEVICE, SYSTEM AND METHOD FOR DETECTING AN ELEMENT IN A FLUID**

(71) Applicant: SENSING SOLUTIONS, S.L., 08193 Cerdanyola del Vallés Barcelona (ES)
(72) Inventor: Gómez Montes, Silvia, 08290 Cerdanyola del Vallés (ES); Collado Sebastià, Marck, 08290 Cerdanyola del Valles (ES)
(74) Representative: Clarke, Modet y Cía., S.L.

(57) **Abstract**

Monitoring device, system and method for detecting an element in a fluid. The monitoring device (1) comprises a particle sensor (139), a VOC sensor (132) that includes a compartment (131), a lens (133) and a laser emitter (135) and a concave mirror (134) optically coupled with the lens (133). The particle sensor (139) electrostatically filters particles in a fluid sample (101b), provides a filtered fluid sample (101a) to the compartment (131), and provides particle data in the fluid sample. The concave mirror (134) concentrates laser light refracted by the filtered fluid sample (101a) in the compartment (131) and directs said concentrated laser light to a measurement area (132a) of the VOC sensor (132). The VOC sensor (132) collects spectrophotometric data (102a) based on Raman spectrography on the filtered fluid sample (101a) from the compartment (131).

## Description

### TECHNICAL FIELD

The present invention refers to techniques for monitoring a fluid, typically air or water, but also applicable to others such as urine, plasma, etc. It falls within the health and/or industrial sectors. It is capable of detecting the presence of multiple elements, particularly volatile organic compounds generated due to the metabolism of bacteria, fungi, viruses, etc. It can be applied to identify a disease, not only infectious but cancer or others where the human or animal body emits distinctive VOCs.

### BACKGROUND OF THE INVENTION

Air constitutes the fastest medium for the rapid and global spread of many types of microorganisms. Microorganisms have adapted to favor their survival and dispersion in the air through sprays, dust particles, dry leaf fragments, water droplets and even droplets of saliva expelled when coughing or speaking. In this way, microorganisms can travel long distances, allowing the disease transmission.

Therefore, in parallel to the development of effective pharmacological treatments against these infectious diseases, the health sector is developing methods for detecting microorganisms in environmental sources for the control and reduction of these infectious diseases.

Numerous standardized methods for the microbiological evaluation of air quality are already known (ISO 14698-1: 2003). Two types can be distinguished: passive and active methods. Passive methods typically use Petri dishes with a solid culture medium that is exposed to the environment for a predefined period of time. Active methods use a forced air flow that hits or passes through a culture medium. In both types, the culture media are incubated under optimal growth conditions according to the type of microorganisms investigated, for a time that usually ranges between 24 and 72 hours. The count of colony-forming units (CFU) in the culture media allows obtaining the quantification of the microbiological load. However, pathogen identification, antibiotic profiling, and detection of specific gene expression require additional investigation, including molecular biology techniques such as next-generation sequencing, rDNA PCR, or enzyme immunoassays.

Many of the systems currently used are not fully automated and contain manual or semi-automated steps that may be subject to human errors.

There are also devices that can detect and evaluate microbial biomass in the air, but they cannot identify the type of biological material detected. On the other hand, there are also some commercially available devices for the detection and quantification of specific bacterial species through automatic sampling of air samples combined with a laser optical detection system.

Patent documents describing microorganism detection systems are also known in the state of the art.

It is worth highlighting Patent US11460407 B2, which describes a method and apparatus for analyzing gas quality, through a Raman spectroscopic examination of a gas sample from a space to be monitored. Spectroscopic examination produces a measurement spectrum that extends over a wavelength range. A deviation of the measurement spectrum of at least one comparison sample is then detected. Based on the deviation detected, a gas quality warning is produced. However, this proposal is intended for the analysis of some environmental indicator such as temperature, pressure or relative humidity.

On the other hand, International Patent Application WO2021061330A1 describes a system and method for providing rapid and autonomous detection of analyte particles in gas and liquid samples using MALDI-MS (matrix-assisted laser desorption/ionization and mass spectrometry) and aerosol chemical analytes using LDI and MALDI-MS using time-of-flight mass spectrometry (TOFMS). In relation to the method of this Patent Application, it should be noted that it is an indirect detection method that requires the use of a disk to collect the sample that will be subsequently processed, obtaining the result after a few days of analysis.

Patent Application CN114858779A describes an gas detection smart method and device, and the method comprises the following steps: providing a microfluidic channel and arranging a SERS substrate in the microfluidic channel; continuously introducing gas to be detected into the microfluidic channel; detecting gas to be detected in the microfluidic channel by adopting Raman detection equipment; performing Raman spectrum analysis according to the detected Raman spectrum to obtain the components and their concentration in the analyzed gas.

All the proposals described above vary in size, detection, portability, sample collection method, use of bioanalytes and ability to detect different microorganisms. None of the existing proposals can be easily adapted for use in both portable and stationary detection. Further, they are quite expensive, which limits their accessibility. Finally, most are not equipped with smart technology interface.

For this reason, there is a need to develop new systems and methods for detecting bacteria in air that are automatic, reliable and in real time.

### BRIEF DESCRIPTION OF THE INVENTION

The invention relates to a monitoring device according to claim 1 and a detection system according to claim 7. It also refers to a detection method according to claim 11, which includes the steps and operations carried out by the aforementioned system.

The invention allows the detection of an element present in a fluid sample that, for example, is associated with bacteria, fungi, or gases of certain metabolites, among others.

The invention uses Raman spectroscopy and *machine learning* (ML)-based processing and neural networks. It allows not only to identify but also to quantify an element.

The objective element of detection through the emission of volatile organic compounds (VOCs) associated with a disease or the metabolism of a microorganism, typically is a bacteria, a fungus or a virus. However, it can also be configured to detect inert elements such as harmful gas emissions.

The applications can be numerous. It is enough to establish an association between the VOC detected and a cause. Therefore, it can be used to detect various circumstances, such as diseases associated with bacteria, fungi and viruses. Also for certain tumors.

Thus, the fluid is generally air or water, although it can be used for a non-pretreated sample, such as a blood, plasma, urine and/or stool sample.

In the context of the present invention, volatile organic compounds (VOCs) are understood to be low molecular weight molecules. There are VOCs excreted by microorganisms as a result of their own metabolism and that usually allow them to establish interactions, beneficial or harmful, with each other and/or with other organisms. These VOCs vary. There are VOCs produced by tumor cells or infected by other microorganisms (bacteria, viruses and/or fungi).

Briefly, Raman spectroscopy is an analysis technique based on the inelastic scattering of monochromatic light (typically a laser). The frequency of photons in monochromatic light changes when interacting with a sample. The photons are absorbed by the sample and then re-emitted. The frequency of the re-emitted photons is shifted up or down compared to the original monochromatic frequency, called the Raman effect, allowing the sample to be characterized.

Generally speaking, the monitoring device to which the invention refers includes a particle sensor, a VOC sensor that has a compartment with an inlet and an outlet so that a fluid can flow, a lens and a laser emitter. A concave mirror is optically coupled with the lens to concentrate light to a measurement area of the VOC sensor. In use, the particle sensor electrostatically filters particles in a fluid sample, provides a filtered fluid sample to the VOC sensor compartment, and additionally provides particle data present in the fluid sample. The VOC sensor obtains spectrophotometric data based on Raman spectrography on the filtered fluid sample from the compartment.

Preferably, the monitoring device can incorporate additional sensors and be portable. Specifically, a carbon dioxide sensor is advantageous, since a high concentration of carbon dioxide in the fluid sample can be indicative of problems (e. g., infection).

Preferably, the monitoring device incorporates data processing capacity through a processing unit (a microprocessor and/or a microcontroller), (e. g., spectrophotometric data). It can also incorporate a communication unit to send a message with the processed data to other devices.

Preferably, the processing unit uses spectrophotometric data with additional particle data and/or carbon dioxide data to apply *machine learning* algorithms to a previously trained neural network. In this way, the device itself can autonomously produce information about the presence and/or quantity of a certain element in the fluid sample. The fluid can come from the environment or from a patient.

Generally, the system for detecting an element in a fluid to which this invention relates includes one or more monitoring devices as described above, and also a server to perform most of the processing tasks and operations, mainly with data from each monitoring device and applying *machine learning* classification algorithms on a previously trained neural network. In this way, the system can produce information about the presence and/or quantity of an element in a fluid sample monitored by a certain monitoring device. Usually, an organism or microorganism whose metabolism emits gases associated with the presence of a fungus, a bacteria or a virus can be detected. Also, a certain harmful gas without being associated with a biological process can be detected.

Generally, a method of detecting an element in a fluid comprises electrostatically filtering a particle sample of the fluid, depositing the particles to obtain a filtered sample and particle data in the fluid; applying Raman spectrography on the filtered sample and obtaining a set of spectrophotometric data; processing the spectrophotometric data set by applying one or more classification algorithms in a trained neural network and obtaining information about the presence of one or more elements in the sample; producing detection information and generating a warning message with information about the presence of one or more elements.

### BRIEF DESCRIPTION OF THE DRAWINGS

To complement the description that is being made and in order to help a better understanding of the features of the invention, a set of drawings is attached as an integral part of said description, where, with an illustrative and non-limiting nature, the following has been represented:
FIGURE 1A: Possible high-level architecture of the detection system.
FIGURE 1B: High-level flowchart of the detection method.
FIGURE 2A: Diagram of a configuration with a concave mirror optically coupled with the lens and a measurement area in a VOC sensor of a monitoring device.
FIGURE 2B: Diagram of the fluid circulation path in a monitoring device.
FIGURES 3A-3B: Diagram of the filtering effect in a compartment of a monitoring device similar to that in FIGURE 2.
FIGURE 3C: Graph example with two Raman spectrum curves.
FIGURE 4: Diagram of an assembly or apparatus for collecting data with which a neural network for use in the subsequent classification of microorganisms is trained.

### DETAILED DESCRIPTION OF THE INVENTION

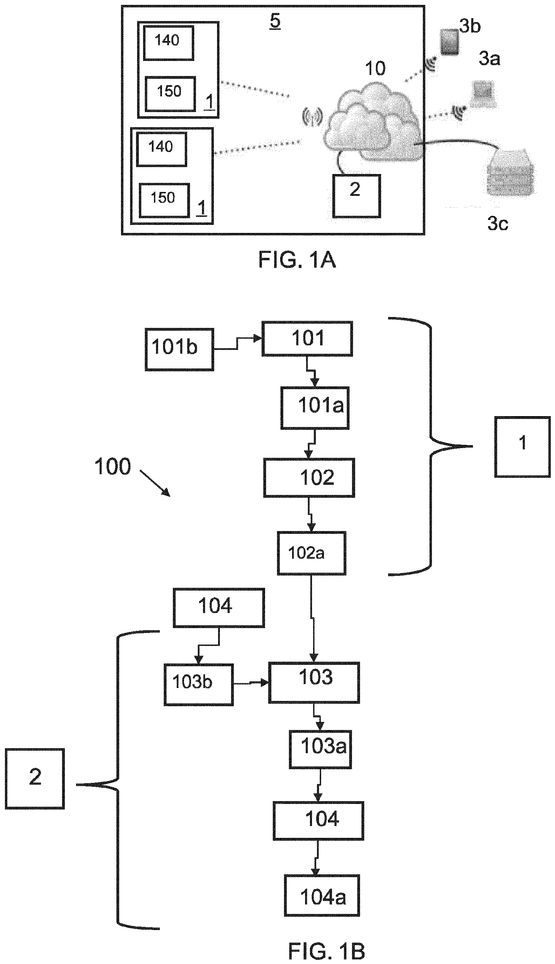

**FIGURE 1A** schematically illustrates a very high level block diagram of a detection system **5** according to an embodiment of the invention. The detection system **5** includes a server **2** and monitoring devices 1 that may be portable and equipped with their own battery, ready to be deployed in various locations from which they can communicate monitoring data (wirelessly or not) through a communications network **10** (Internet, 3/4/5G, etc.). Each monitoring device **1** includes a processing unit **150** and communication unit **140** for sending a message to the server **2,** including, in other embodiments, to a user device, such as a mobile phone **3b,** or a computer **3a.**

The monitoring data (mainly including spectrophotometric data and, additionally, CO2 data, particles, etc.) are received by server **2,** which processes and classifies them to identify if they correspond to any element by applying *machine learning* (ML) algorithms on a neural network previously trained with examples. The server **2** of the detection system **5** can be configured to send information related to the detection, either to another external server **3c** (for example, from a hospital computer system), or to user devices **3b, 3a,** typically associated with supervision or surveillance staff.

**FIGURE 1B** schematically illustrates a flowchart with the main steps of the detection method **100,** which comprises a sequence with the following steps:
- Electrostatic filtering step **101** for filtering particles from a sample **101b,** depositing them. A filtered sample **101a** and data on the particles present in the fluid are obtained. Particles serve as a vehicle for the transport of elements (e. g., bacteria).
- Raman spectrography applying step **102** on the filtered sample **101a.** Spectrophotometric data **102a** are obtained.
- Step **103** for processing the spectrophotometric data **102a** by applying one or more ML algorithms on the trained neural network **103b.** Information is obtained about the presence of one or more elements **103a** in the sample. Along with the spectrophotometric data, CO2 and particle data in the fluid can be acquired.
- Step for producing detection information **104.** A warning message **104a** is obtained with information about the presence of one or more elements **103a.**

In a particular application, method **100** allows the presence of volatile organic compounds (VOCs) produced by bacteria, viruses or fungi to be determined in an ambient air sample. In other applications, diseases (cancer) where the patient emits specific gaseous metabolites, bacterial or viral infections of a patient, can be detected. Even an element not related to bacteria, viruses or fungi such as complex gas leaks such as anesthetic gases or explosive gases. In patient applications, the monitoring device **1** is adapted to collect a sample of air exhaled by a patient (e. g. through a mouthpiece or an adapter for a respirator).

It is clarified that in the proposed detection method **100,** the usual Raman pattern recognition techniques are not used and that the spectrum of all the gases present in the sample is obtained. Therefore, there is no pretreatment to remove certain gas spectra. A sample **101b** is analyzed without subtracting a blank.

In a particular embodiment, data acquisition is carried out with the monitoring device 1, while the processing and detection, mainly, is carried out with the server **2** (see FIGURE 1A). Thus, the electrostatic filtering step **101** and the Raman spectrography applying step **102** are carried out by the monitoring device **1.** Similarly, the processing step **103** and the detection information producing step **104** are carried out by the server **2.**

In another particular embodiment, at least part of the tasks associated with server **2** can be transferred to monitoring device **1.**

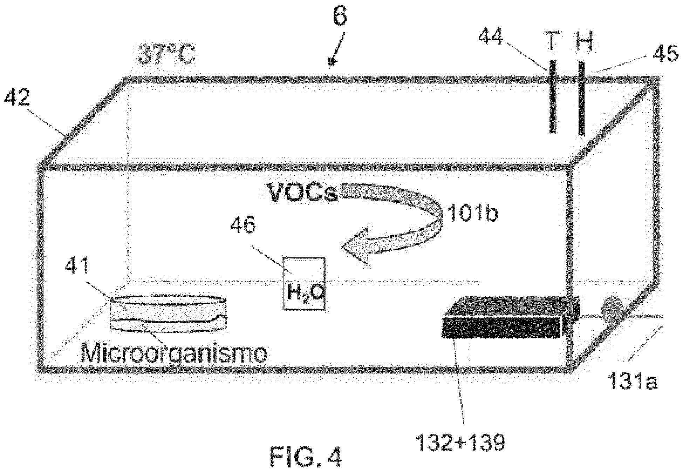

Preferably, in the acquisition, to improve the results of the ML analysis, in addition to using the spectrum, particle and CO2 data is included and to train the neural network with the spectrum and additional parameters. This trained neural network **103b** is the result of a training step **90** previously carried out (see FIGURE 4). Additionally, it can be continuously retrained with current data acquired by the monitoring device **1.**

During the (previous) training step **90,** the neural network is trained with various ML algorithms, the one that provides the best performance is determined, and said algorithm is chosen to retrain the neural network. These testing, selection and retraining steps are repeated dynamically and iteratively and can also be applied to current data acquired by each monitoring device **1.**

It has been observed that the emission of different bacteria is sometimes similar but that, when introducing a new bacteria, the algorithm is no longer the most appropriate and an another one more complex must be used to differentiate between more bacteria. In these cases, it is advantageous to change the algorithm and configuration of the neural network. Preferably, it is a programmed process that runs automatically, typically on server **2** of detection system **5.**

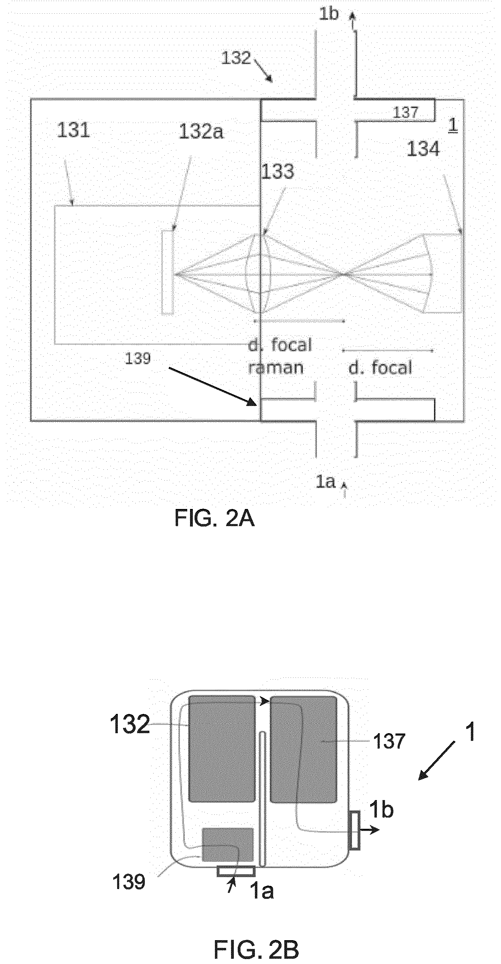

**FIGURE 2A** illustrates some components of an embodiment of the monitoring device 1 in a fluid (e. g., air) where part of the components for data acquisition can be seen. A compartment **131** is shown where a VOC sensor **132** is located, which can preferably be carried out with a Raman spectrophotometer. The VOC sensor **132** has a laser emitter (not shown in this figure) and a lens **133.** The VOC sensor **132** has an associated particle detector **139** that also acts as a particle sensor to improve the acquisition of spectrophotometric data by filtering particles from the fluid that passes into compartment **131.** Also, CO2 data is obtained with a CO2 sensor **137.**

A concave mirror **134** is incorporated after the lens **133** of the VOC sensor **132** to increase the signal-to-noise ratio and obtain better readings. The concave mirror **134** allows the light not reflected by the molecules and refracted to be collected. Thus, the light is concentrated in the measurement area of the VOC sensor **132.** To concentrate it correctly in the measurement area, the degree of curvature of the concave mirror **134** must be calculated from its focal distance. Typically, this measurement area is a CMOS sensor circuit **132a** in the case of a Raman spectrophotometer, so the focal distance of the concave mirror **134** should be the same or very similar to that of the lens **133.**

**FIGURE 2B** illustrates with arrows the circulation path of a fluid in an embodiment of the monitoring device **1.** The fluid (e. g., air) enters through an inlet **1a,** absorbed by a small fan (not shown). Usually, the fan itself of the particle sensor **139** coupled with input **1a** is used. The fluid filtered by the particle sensor **139** then passes to the VOC sensor **132** (which may incorporate the concave mirror already indicated in FIGURE 2A). After which, the fluid can pass through a CO2 sensor **137** or another additional sensors until it leaves through an outlet **1b.**

In another embodiment of the monitoring device **1,** air from a respirator can be collected. In that case, an adapter is used to connect input **1a** to the output of said respirator and the fan can be dispensed with.

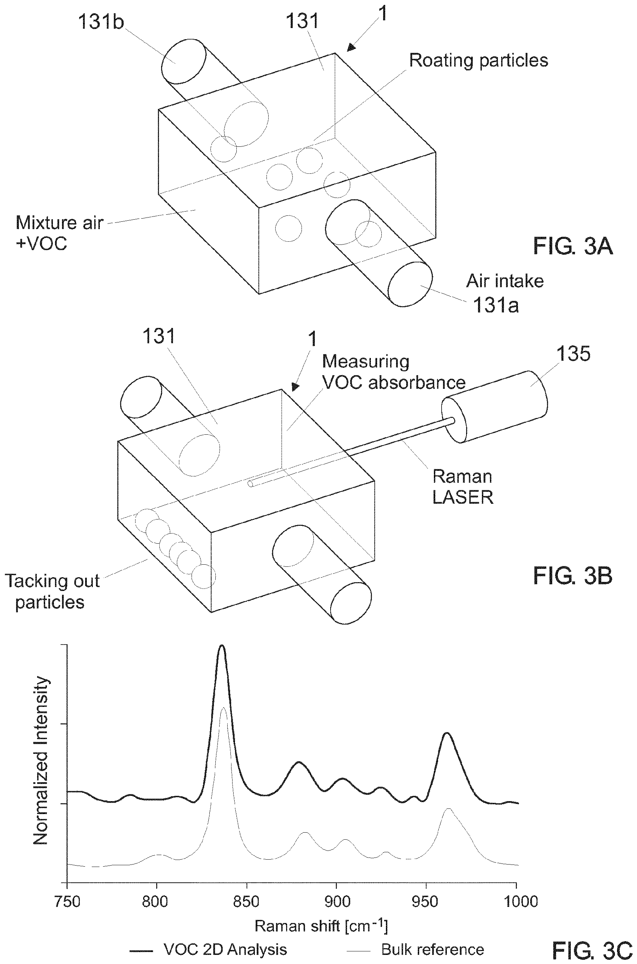

**FIGURES 3A-3B** show the filtering effect in compartment **131** of an air monitoring device **1,** where is previously illustrated (FIGURE 3A) and subsequently (FIGURE 3B) to electrostatic filtering.

To acquire air without interference from particles, the air is passed through compartment **131,** and electrostatic deposition is applied to remove the particles present in the air that arrives from an inlet **132a** and leaves outlet **132b** by positive pressure from compartment **131** the filtered air. This filtering can be done with a particle sensor integrated with the VOC sensor **131.** In this way, the laser emitter **135** associated with the VOC sensor does not interact with particles already filtered.

**FIGURE 3C** shows a graphic representation where the normalized intensity is represented on the ordinate axis; on the abscissa axis, the Raman shift expressed in wave numbers (cm-1). The upper curve corresponds to a spectrum with Raman spectrophotometric data from the VOC sensor present in a sample and the lower curve is the *bulk* reference. Generally, thousands of data are acquired per second.

**FIGURE 4** illustrates a support assembly **6** to obtain the VOCs released by a controlled colony of a certain microorganism and its quantity (or a mixture of microorganisms that are capable of cohabiting with their respective CFUs).

The assembly **6** includes a Petri dish **41** or similar to facilitate the growth of the microorganism **101b,** includes a water container **46** to maintain humidity, a temperature sensor **44** and a humidity sensor **45** to facilitate the control of environmental conditions according to the type of microorganism.

The assembly **6** further includes a VOC sensor **132** to acquire CFU quantity, Raman spectrophotometric data, and optionally the assembly **6** can acquire particle data and CO2 data. The above components are located within a chamber **42** (e. g., with dimensions 70cm x 25cm x 30cm).

Spectrophotometric, particle and CO2 data are labeled and associated with a certain element (e. g. a certain bacteria) and are used as examples to train a neural network with which to subsequently identify samples in the environment or in a patient. In this way, assembly **6** serves to acquire data with which, properly labeled, a training stage of the neural network can be carried out (see FIGURE 1B) in a computer. Once the neural network is trained on the computer, it can be transferred to another equipment (for example, to the monitoring device, or more usually to the detection system server; see FIGURE 1A).

To not only identify but also quantify an element (e. g. a microorganism), a database of pathogenic bacteria is used, for example, from NCBI and the database of human pathogens based on the classification of airborne bacteria through high yield sequencing of 16S rRNA to obtain the relationship between PM 2. 5 and PM 10 with respect to bacterial families. In addition, other parameters that affect the growth of colonies are taken into account with which to obtain information on their quantity.

Throughout the description and claims, the word "comprises" and its variations are not intended to exclude other features, additives, components or technical steps. Additional objects, advantages and features of the invention will become apparent to those skilled in the art upon examination of the description or may be learned by practicing the invention. Furthermore, the present invention encompasses all possible combinations of particular and preferred embodiments described herein.

## Claims

1. Fluid monitoring device (1) comprising:
- a particle sensor (139);
- a volatile organic compound (VOC) sensor (132) comprising a compartment (131) that allows the passage of fluid, a lens (133) and a laser emitter (135);
- a concave mirror (134);
wherein the particle sensor (139) is configured to electrostatically filter particles in a sample of the fluid (101b), provide a filtered fluid sample (101a) to the compartment (131) and obtain particle data in the fluid sample;
where the concave mirror (134) is optically coupled with the lens (133) of the VOC sensor (132) to concentrate laser light refracted by the filtered fluid sample (101a) in the compartment (131) and direct said concentrated laser light to a measurement area (132a) of the VOC sensor (132);
where the VOC sensor (132) is configured to collect spectrophotometric data (102a) based on Raman spectrography in the filtered fluid sample (101a) from the compartment (131).

2. Monitoring device (1) according to claim 1, further comprising a processing unit (150) configured to process the spectrophotometric data (102a) and a communication unit (140) configured to send a message with the spectrophotometric data (102a) to a user device (3a, 3b) and/or to a server (2, 3c).

3. Monitoring device (1) according to claim 2, further comprising a carbon dioxide sensor (137) for collecting carbon dioxide data in a fluid sample.

4. Monitoring device (1) according to claim 3, further comprising an internal fan, an inlet (1a) for introducing fluid from the outside and an outlet (1b) for returning the introduced fluid to the outside, after having first circulated through the particle sensor (139), followed by the VOC sensor (132) and subsequently by the carbon dioxide sensor (137).

5. Monitoring device (1) according to any one of claims 1 to 4, comprising a battery for supplying electrical energy.

6. Monitoring device (1) according to any one of claims 2 to 5, wherein the processing unit (150) is configured to process the spectrophotometric data (102a) and additional particle data and/or carbon dioxide data and apply one or more *machine learning* algorithms to a previously trained neural network, and to produce information about the presence and/or quantity of an element in the fluid sample, where the element is at least one of the following: a fungus, a bacteria, a virus, a harmful gas, or a gas associated with a tumor.

7. System (5) for detecting an element in a fluid comprising:
- one or more portable monitoring devices (1) according to any one of claims 1 to 6;
- a server (2) configured to process the spectrophotometric data (102a) and apply one or more *machine learning* algorithms to a previously trained neural network and to produce information about the presence and/or quantity of an element in a fluid sample in a particular monitoring device (1), where the element is at least one of the following: a fungus, a bacteria, a virus or a harmful gas.

8. Detection system (5) according to claim 7, where the elements are living microorganisms equipped with a gas-emitting metabolism.

9. Detection system (5) according to claim 7 or 8, wherein in addition to spectrophotometric data (102a) the monitoring device (1) provides additional particle and carbon dioxide data that is processed by the server (2) together with the spectrophotometric data (102a).

10. Detection system (5) according to any one of claims 7 to 9, wherein the server (2) is configured to test the neural network by applying a plurality of algorithms, select one based on the performance obtained and retrain said neural network with the algorithm selected.

11. Method for detecting an element in a fluid comprising:
- electrostatically filtering a sample (101b) of the fluid from particles, depositing the particles to obtain a filtered sample (101a) and particle data in the fluid;
- applying Raman spectrography on the filtered sample (101a) and obtaining a set of spectrophotometric data (102a);
- processing the set of spectrophotometric data (102a) by applying one or more classification algorithms in a trained neural network (103b) and obtaining information about the presence of one or more elements (103a) in the sample;
- producing detection information (104) and generating a warning message (104a) with information about the presence of one or more elements (103a).
